# EUROPÄISCHE PATENTANMELDUNG

(11) **EP 4 606 333 A1**
(43) Veröffentlichungstag der Anmeldung: **27.08.2025**
(21) Anmeldenummer: 24159496.9
(22) Anmeldetag: 23.02.2024
(51) Int. Cl.: A61B 18/14, A61B 18/00

(54) **ELEKTROCHIRURGISCHES INSTRUMENT MIT KOHLENSTOFFFASERELEKTRODE**

(71) Anmelder: Erbe Elektromedizin GmbH, 72072 Tübingen (DE)
(72) Erfinder: Schnitzler, Uwe, 72074 Tuebingen (DE); Sadler, Daniel, 72074 Tuebingen (DE)
(74) Vertreter: Rüger Abel Patentanwälte PartGmbB

(57) **Zusammenfassung**

Bei einem erfindungsgemäßen Instrument (15) zur chirurgischen Behandlung einer Gewebeoberfläche (14) insbesondere zur Koagulation oder Ablation derselben, dient eine aus Kohlenstofffasern (29) bestehende oder wenigstens Kohlenstofffasern (29) aufweisende Elektrode (23) zur Stromzuleitung zu der Gewebeoberfläche (14). Infolge der Anisotropie der elektrischen Leitfähigkeit der Kohlenstofffasern (29) oder der ausgebildeten Elektrode (23) kann eine großflächige und gleichmäßige Stromverteilung erreicht werden. Und zwar dies sowohl in direkter Kontaktkoagulation, als auch in gemischter Koagulation bei Plasmaausbildung unter zumindest teilweiser Berührung der Gewebeoberfläche (14) durch die Elektrode (23). Durch die Ausbildung der Elektrodenoberfläche durch Kohlenstofffasern (29) und insbesondere deren hoher Wärmeleitfähigkeit wird ein Anhaften der Elektrode (23) an der Gewebeoberfläche (14) wirksam unterbunden.

## Beschreibung

Die Erfindung betrifft ein Instrument zur elektrochirurgischen Behandlung lebenden Gewebes von menschlichen oder tierischen Patienten. Insbesondere betrifft die Erfindung ein Instrument zur oberflächlichen Koagulation solchen Gewebes.

Zur Behandlung, insbesondere Koagulation und/oder Ablation, von lebendem Gewebe sind Instrumente zur Argo-Plasma-Koagulation bekannt. Ein solches Instrument kann beispielsweise der WO 2021/013852 A1 entnommen werden. Das dort beschriebene Instrument ist als flexible Sonde ausgebildet und weist einen Schlauch mit einem gasführenden Lumen auf. In der Nähe der distalen Gasauslassöffnung des Schlauches ist eine Elektrode angeordnet, die über eine elektrische Zuleitung mit einem Generator verbunden ist, der eine zur Plasmabildung ausreichend hohe Spannung an die Elektrode liefert. Der das Lumen durchfließende Argonstrom wird an der Elektrode ionisiert und tritt als Plasmastrahl in distaler Richtung aus dem Instrument aus.

Mit einem solchen Instrument lassen sich ausgewählte Gewebebereiche präzise und abgegrenzt behandeln.

Es besteht allerdings gelegentlich der Wunsch nach einer großflächigeren Gewebebehandlung, beispielsweise bei der Mucosa-Ablation im Magen. Dort kann die Notwendigkeit bestehen, große Areale, zum Beispiel bis zu zwei Drittel der Magenoberfläche, elektrochirurgisch mittels Argon-Plasma-Koagulation zu behandeln.

Dazu offenbaren die EP 3 141 203 B1 und die EP 3 141 204 B1 jeweils ein Ablationsinstrument, das zur großflächigen Mucosa-Ablation geeignet ist. Das Instrument weist einen Sondenschlauch auf, der an seinem distalen Ende einen Kopf trägt, in dem sich das Lumen des Schlauchs zunächst fortsetzt und dann gabelt. Das Lumen mündet so in zwei voneinander getrennten Austrittsöffnungen. In diesen sitzen Elektroden, die jeweils einen Plasmastrahl erzeugen. Die beiden erzeugten Plasmastrahlen treffen nebeneinander auf die Gewebeoberfläche auf. Auf diese Weise wird ein breiterer Ablationsstreifen erzeugt.

Ein weiteres Instrument zur Argon-Plasma-Koagulation ist aus der DE 195 35 811 C1 bekannt. Dieses Instrument weist ein Rohr auf, in dessen Ende eine Düse eingesetzt ist. In einer Variante weist die Düse an ihrer Austrittsöffnung einen Block aus gesinterten Kügelchen aus isolierendem Keramikmaterial oder auch aus elektrisch leitfähigem Material, wie zum Beispiel Metall oder Kohlenstoff auf. In diesem Fall wird empfohlen, die Düse nochmals in eine isolierende Hülse einzusetzen, um eine direkte Berührung mit Gewebe zu verhindern.

Weiter sind Instrumente zur berührenden Gewebekoagulation bekannt, bei denen eine Koagulationselektrode in direkten Kontakt mit lebendem Gewebe gebracht wird. Dazu offenbart die US 4,074,718 B1 ein Instrument mit einer kohlenstoffbeschichteten Elektrode. Die Kohlenstoffbeschichtung soll ein Ankleben der Elektrode am Gewebe verhindern.

Auch die US 2013/0110105 A1 offenbart ein elektrochirurgisches Instrument mit einer distalen Elektrode, die eine Kunststoffbeschichtung trägt.

Weiterer Stand der Technik ist aus der WO 2014/197632 A2**,** der CN 11 683 088 A, der US 2012/083782 A1**,** der US 2016/121134 A1**,** der EP 3 708 222 A1 bekannt.

Während je nach Durchmesser und Größe des Plasmastrahls ein abgegrenzter Bereich des Gewebes behandelt werden kann, ist mit der Kontaktkoagulation eine noch gezieltere lokale Behandlung scharf abgegrenzter Bereich möglich.

Es ist Aufgabe der Erfindung, ein elektrochirurgisches Instrument zu schaffen, mit dem sich ausgedehnte Flächenbereiche biologischen Gewebes schnell und gleichmäßig oberflächlich koagulieren lassen.

Diese Aufgabe wird mit im Instrument nach Anspruch 1 gelöst:
Das erfindungsgemäße Instrument weist einen Schaft auf, der an seinem distalen Ende eine Elektrode aufweist, die über eine Leitung und einen elektrischen Generator mit Behandlungsstrom versorgbar ist. Die Elektrode weist Kohlenstofffasern auf, die elektrisch leitfähig sind. Die Kohlenstofffasern bedingen die elektrische Leitfähigkeit der Elektrode und sorgen zugleich zu einer großflächigen Stromverteilung, wenn die Elektrode entsprechend ihrer eigenen räumlichen Ausdehnung einen grö-ßeren Flächenbereich des biologischen Gewebes berührt. Die an dem länglichen Schaft befestigte Elektrode kann dabei, insbesondere quer zur Schaftlängsrichtung gemessen, eine Breite aufweisen, die größer ist als die Breite des Schafts. Die Kohlenstofffasern bilden dabei einzelne widerstandsbehaftete linienhafte Leiter, die jeder für sich elektrischen Strom leiten und relativ gleichmäßig auf die Fläche verteilen auf der Berührung zwischen der Elektrode und dem Gewebe besteht.

Der längliche Schaft kann ein steifer oder flexibler Schaft sein, der an seinem distalen Ende die Elektrode trägt und an seinem proximalen Ende Mittel zum Anschluss der elektrischen Leitung an einen Generator aufweist. Die Kohlenstofffasern aufweisende Elektrode dient dann vorwiegend zur Kontaktkoagulation.

Es ist auch möglich, in dem länglichen Schaft ein oder mehrere Lumen vorzusehen, sodass der Schaft als Schlauch oder Rohr ausgebildet ist. Das Lumen kann mittels geeigneter Anschlussmittel an eine Gasversorgungsquelle angeschlossen oder anschließbar sein. Sind mehrere Lumen vorhanden können diese an ein und dieselbe oder auch an unterschiedliche Gasquellen angeschlossen sein.

An dem distalen Ende des Schlauchs (oder Rohrs) kann eine mit dem oder den Lumen in Verbindung stehende Auslassöffnung vorgesehen sein. Aus der Auslassöffnung kann ein geeignetes Gas, insbesondere ein Inertgas, wie beispielsweise Argon, ausgelassen werden. Die Auslassöffnung und die Elektrode sind in Bezug aufeinander vorzugsweise so angeordnet, dass das ausgelassene Gas die Elektrode umspült.

Die Elektrode kann in oder an dem Schaft fest oder beweglich gelagert sein. Ist der Schaft als Schlauch oder Rohr ausgebildet, kann die Elektrode zum Beispiel ganz oder teilweise in dem Lumen oder außerhalb des Lumens vor der Auslassöffnung angeordnet sein. Insbesondere kann die Elektrode aus dem Lumen herausschiebbar beweglich angeordnet sein. Die Elektrode kann sowohl zur Ionisierung eines Gasstroms und somit zur Plasmaerzeugung wie auch gleichzeitig oder alternativ zur Kontaktkoagulation von der zu behandelnden Gewebefläche dienen. Insbesondere wenn die Elektrode einen ohmschen Widerstand aufweist, der größer ist als die Behandlungsspannung geteilt durch maximalen Behandlungsstrom wirkt die Elektrode für den zur Kontaktkoagulation dienenden Strom strombegrenzend und gestattet deswegen weiterhin die Erzeugung von Plasma zur Koagulation des Gewebes. Dies kann dazu beitragen, eine gleichmäßige und großflächige und auch rasche Koagulation des Gewebes zu erreichen.

Die aus Kohlenstofffasern bestehende oder Kohlenstofffasern enthaltende Elektrode kann außerdem eine Vorzugsrichtung für die Stromleitung aufweisen, wobei die Vorzugsrichtung durch Kohlenstofffasern bestimmt ist. Auch dies kann zur Egalisierung der Stromverteilung über die gesamte Elektrode führen. Z.B. kann eine Elektrode in Elektrodenlängsrichtung einen geringen elektrischen Widerstand aufweisen. Dies kann durch einen sich in Elektrodenlängsrichtung erstreckenden, z.B. mittig in der Elektrode angeordneten Metalldraht gefördert werden. Von dem Metalldraht ausgehend muss der Strom auf seinem Weg zu der Elektrodenoberfläche einen elektrischen Widerstand überwinden, was zu einer Vermeidung oder Verringerung lokaler Stromdichtespitzen auf der Elektrodenoberfläche beitragen kann.

Die Elektrode kann in oder an der Auslassöffnung des Lumens beweglich angeordnet sein. Sie kann zum Beispiel in einer ersten Position in dem Lumen angeordnet sein, während sie sich in einer zweiten Position ganz oder teilweise außerhalb des Lumens befindet. Auf diese Weise kann das Instrument, je nach Position der Elektrode, verschiedene Koagulationsarten bevorzugen. Beispielsweise kann das Instrument zu einer Plasmakoagulation dienen, solange die Elektrode innerhalb des Lumens positioniert ist. Steht die Elektrode hingegen ganz oder teilweise außerhalb des Lumens, kann zu der Plasmakoagulation eine Kontaktkoagulation hinzutreten oder auch bei großflächigen Gewebekontakt der Elektrode die Kontaktkoagulation vorherrschen oder allein wirksam sein.

Die Elektrode kann steif oder flexibel, zum Beispiel als Pinsel, Schlinge, Kordel oder steifer oder flexibler Spatel, ausgebildet sein. Die Kohlenstofffasern können in einen Festkörper eingebunden sein, zum Beispiel in eine Kunststoffmatrix. Die Kunststoffmatrix kann aus einem elektrisch isolierenden Kunststoff ausgebildet sein. Damit bestimmen die Anzahl und Anordnung der Kohlenstofffasern die Leitfähigkeit und die bevorzugte Stromflussrichtung in der Elektrode sowie letztlich auch die Stromverteilung an der Elektrodenoberfläche. Es ist aber auch möglich, für die Kunststoffmatrix einen intrinsisch oder extrinsisch leitfähigen Kunststoff zu verwenden.

Die Kohlenstoffasern können vollständig in den Körper der Elektrode eingebunden sein oder aus dem Körper herausragende Enden aufweisen. Ebenso können die Kohlenstofffasern durch Flechten, Verdrillen oder andere Maßnahmen zu einem Faden oder einer Kordel zusammengefasst sein, die an einem Ende oder an ihren beiden Enden mit der Stromzuführungsleitung verbunden ist. Auch ist es möglich, die Kohlenstofffasern alle jeweils an einem Ende in einer Fassung zu halten, während die anderen Enden nach Art eines Pinsels aus dieser Fassung heraus und von dieser abstehen. Es ist möglich, zusätzlich zu den Kohlenstofffasern in der Elektrode anderweitige elektrische Leiter, zum Beispiel einen blanken Metalldraht oder dergleichen, anzuordnen, um zum Beispiel die Federeigenschaft einer schlingenförmigen Elektrode oder die elektrischen Eigenschaften derselben gezielt zu beeinflussen.

Weiter ist es möglich, die Flexibilität der Elektrode derart auszulegen, dass die Elektrode einerseits in zusammengedrücktem Zustand das Lumen des Schlauchs oder Rohrs passt und andererseits, wenn sie aus dem Lumen heraus positioniert ist, expandiert und so eine Querabmessung aufweist, die größer ist als die Querabmessung des Lumens.

Weitere Einzelheiten vorteilhafter Ausführungsformen der Erfindung sind Gegenstand von Ansprüchen sowie der Zeichnung und der zugehörigen Beschreibung. In der Zeichnung zeigen:
Figur 1 eine Ablationseinrichtung mit einem erfindungsgemäßen Instrument, ein Gerät zur Versorgung des Instruments sowie biologisches einer thermischen Behandlung unterworfenes Gewebe, in schematisierter Darstellung,
Figur 2 das distale Ende des Instruments nach Figur 1,
Figur 3 das distale Ende einer alternativen Ausführungsform eines erfindungsgemäßen Instruments, in längsgeschnittener Darstellung,
Figur 4 einen Ausschnitt aus der Elektrode des Instruments nach Figur 3,
Figur 5 einen Ausschnitt aus einer alternativen Ausführungsform der Elektrode des Instruments nach Figur 3,
Figur 6 eine alternative Elektrodenform für das Instrument nach Figur 3 mit der Materialstruktur nach Figur 4 oder 5,
Figur 7 das distale Ende des Instruments nach Figur 1, in einer alternativen Bauform mit Elektrode als Kordel in Schlingenform,
Figur 8 das Instrument nach Figur 7 mit in das Lumen des Schafts eingezogener Elektrode,
Figur 9 die Elektrode nach Figur 7 während der Koagulation von biologischem Gewebe mit einer zusätzlichen schematisch vereinfachten Darstellung der elektrischen Verhältnisse,
Figur 10 eine alternative Ausführungsform des erfindungsgemäßen Instruments ohne eigene Gaszufuhr,
Figur 11 das distale Ende des Instruments nach Figur 10 in längsgeschnittener Darstellung und
Figur 12 das distale Ende eines abgewandelten Instruments, in einer alternativen Bauform mit einer Elektrode in doppelter Schlingenform.

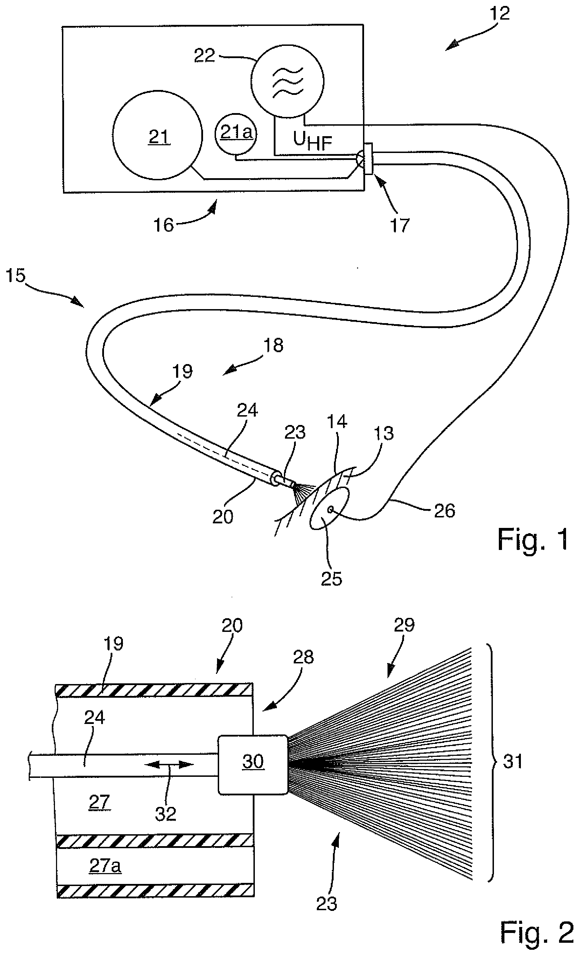

Aus Figur 1 geht eine Behandlungseinrichtung 12 hervor, die zur flächenhaften Koagulation von biologischem Gewebe 13 dient, das in Figur 2 sehr schematisch veranschaulicht ist. Das biologische Gewebe 13 weist eine Oberfläche 14 auf, die beispielsweise von einer inneren Organoberfläche, wie beispielsweise der Magenschleimhaut oder dergleichen gebildet sein kann.

Zu der Einrichtung 12 gehören ein Instrument 15 und ein zur Versorgung des Instruments 15 eingerichtetes Gerät 16, an das ein am proximalen Ende des Instruments 15 vorgesehenes Anschlussmittel, zum Beispiel in Gestalt eines Steckers 17, angeschlossen ist.

Das Instrument 15 weist einen langen, schlanken Schaft 18 auf, der in vorliegendem Fall als flexibler Schlauch 19 oder auch als Rohr ausgebildet ist. Der flexible Schlauch 19 ist dabei dazu eingerichtet, durch den Arbeitskanal eines nicht weiter veranschaulichten Endoskops oder ein anderes, Zugang zu dem Körperinneren eines Patienten bietenden Instrumentariums an den Behandlungsort in Opposition zu der Gewebeoberfläche 14 gebracht zu werden. Während das proximale Ende des Schlauchs 19 an das Gerät 16 außerhalb des Patienten angeschlossen ist, befindet sich das distale Ende 20 des Instruments 15 an oder in dem Patienten.

Das in Figur 1 veranschaulichte Instrument 15 dient der Koagulation der Gewebeoberfläche 14 unter einer Argonatmosphäre. Dazu weist das Gerät 16 eine Gasquelle 21 auf, die dazu eingerichtet ist, das Instrument 15 über den Stecker 17 mit Argon oder auch einem anderen geeigneten Gas zu versorgen. Außerdem enthält das Gerät 16 einen Hochfrequenzgenerator 22, der mit einem Pol an eine zu einer Elektrode 23 des Instruments 15 führende Leitung 24 und mit seinem anderen Pol an eine zu einer Neutralelektrode 25 führende Leitung 26 angeschlossen ist. Die Neutralelektrode 25 ist großflächig an dem Patienten anzubringen.

Wie Figur 2 veranschaulicht, umschließt der Schlauch 19 wenigstens ein Lumen 27, über das das dem Schlauch 19 zugeführte Gas, vorzugsweise Argon, zu der Elektrode 23 fließt und an einer Auslassöffnung 28 ausströmt, die an dem distalen Ende 20 des Schlauchs 19 angeordnet ist. Optional kann der Schlauch 19 ein oder mehrere weitere Lumen 27a aufweisen, die an die gleiche Gasquelle 21 oder an eine oder mehrere weitere Fluidquellen 27a, insbesondere Gasquellen oder auch Flüssigkeitsquellen angeschlossen sein können.

Die Elektrode 23 weist eine Vielzahl von Kohlenstofffasern 29 auf, die zum Beispiel nach Art eines Pinsels angeordnet sein können. Sie weisen dazu ein in einer Fassung 30 gehaltenes Ende auf und erstrecken sich von der Fassung 30 in distaler Richtung von der Fassung 30 und somit von der Öffnung 28 weg. Dabei können die Kohlenstofffasern 29, wie Figur 2 andeutet, von der Fassung 30 divergent wegstehend angeordnet sein, sodass der so gebildete Pinsel 31 eine Breite aufweisen kann, die die den Durchmesser des Lumens 27 übersteigt. Die Breite ist dabei quer zu dem Lumen und somit quer zu der Leitung 24 zu messen. Der Pinsel 31 leitet elektrischen Strom fast ausschließlich in Faserlängsrichtung, wobei jede Faser 29 einen elektrischen Widerstand aufweist. Dadurch führt eine Berührung von einer oder wenigen Kohlenstofffasern zu der Gewebeoberfläche 14 nicht dazu, dass der gesamte von dem Generator gelieferte Strom über diese Fasern abfließen würde. Vielmehr bleibt die Generatorspannung an den übrigen Kohlenstofffasern erhalten.

Die Elektrode 23 kann axial fix oder auch in Längsrichtung des Lumens und somit in Längsrichtung der Leitung 24 beweglich angeordnet sein. Beispielsweise kann die Elektrode 30 durch entsprechende Positionierung entlang des Pfeils 32 (Figur 2) in das Lumen 27 hinein oder aus diesem herausbewegt werden.

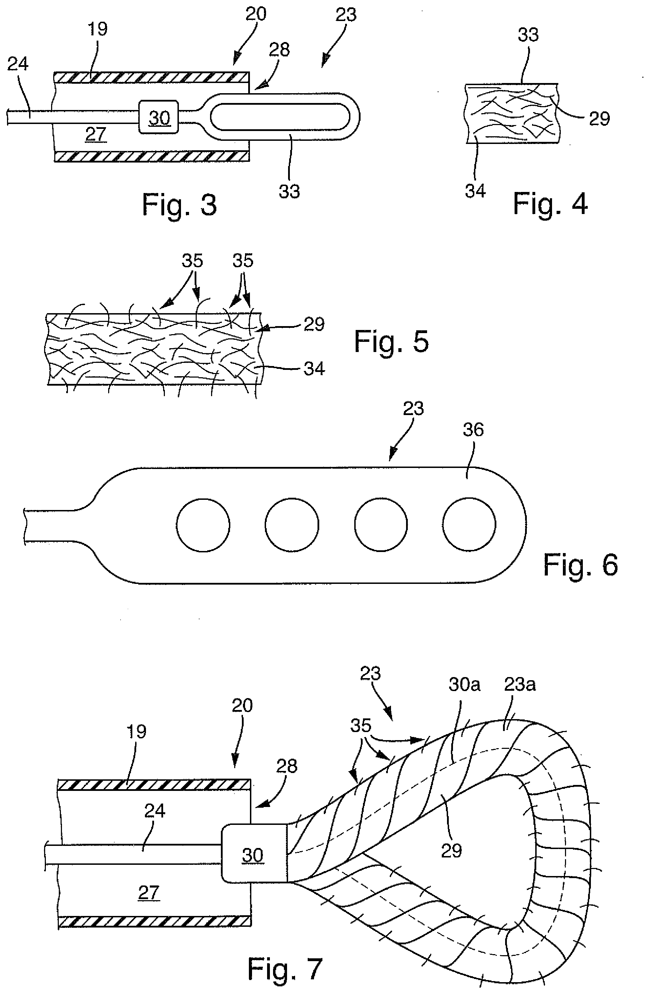

Die Elektrode 23 des Instruments 15 kann anderweitig ausgebildet sein. Figur 3 veranschaulicht dazu eine Ausführungsform, bei der die Elektrode 23 als ovaler, steifer oder wenig flexibler Körper 33 ausgebildet ist. Dieser Körper 33 ist vorzugsweise ein Kunststoffkörper, dessen innere Struktur aus Figur 4 hervorgeht. Der Kunststoffkörper wird durch eine Kunststoffmatrix 34 aus einem elektrisch wenig oder nichtleitenden Kunststoff gebildet, in die zahlreiche Kohlenstofffasern eingelagert sind. Es kann sich dabei sowohl um relativ kurze Fasern, als auch um längere Fasern handeln. Die Kohlenstofffasern 29 können dabei eine Vorzugsrichtung aufweisen oder alternativ auch ohne Vorzugsorientierung angeordnet sein. Die Kohlenstofffasern können vollständig in den Körper 33 eingebettet sein, so dass der Körper 33 eine glatte Oberfläche aufweist. Alternativ können die Kohlenstofffasern 29 aus der Kunststoffmatrix 34 herausragende Enden 35 aufweisen, sodass die Elektrode 23 dann eine raue oder haarige Oberfläche hat.

Die Elektrode 23 ist keineswegs auf die in Figur 3 veranschaulichte Ovalform beschränkt. Sie kann auch als lanzettförmiger oder ovaler Spatel, als Nadel oder Stab oder, wie Figur 6 veranschaulicht, als gelochter Spatel 36 ausgebildet sein.

Bei einer weiter abgewandelten Ausführungsform, die in Figur 7 veranschaulicht ist, sind die Kohlenstofffasern 28 der Elektrode 23 zu einem Seil oder einer Kordel 23a geformt, deren beide Enden in der Fassung 30 gehalten sind. Die Elektrode 23 bildet somit eine flexible weiche Schlinge oder Schleife. Diese enthält miteinander verdrillte oder verseilte Kohlenstofffasern, wobei Enden 35 derselben aus der Kordel 23a oder dem Seil herausschauen können. Falls gewünscht kann die Kordel 23a einen in ihr etwa mittig angeordneten, in Figur 7 durch eine gestrichelte Linie symbolisierten Draht 30a aus Metall oder Kunststoff aufweisen. Dieser (Kunststoff-) Draht 30a kann der Aussteifung der Kordel 23a dienen. Wenn er aus Metall ausgebildet ist oder wenn der Kunststoffdraht mit einem Metalldraht kombiniert ist, kann er zusätzlich eine Vergleichmäßigung der Stromverteilung an der Kordel 23a dienen.

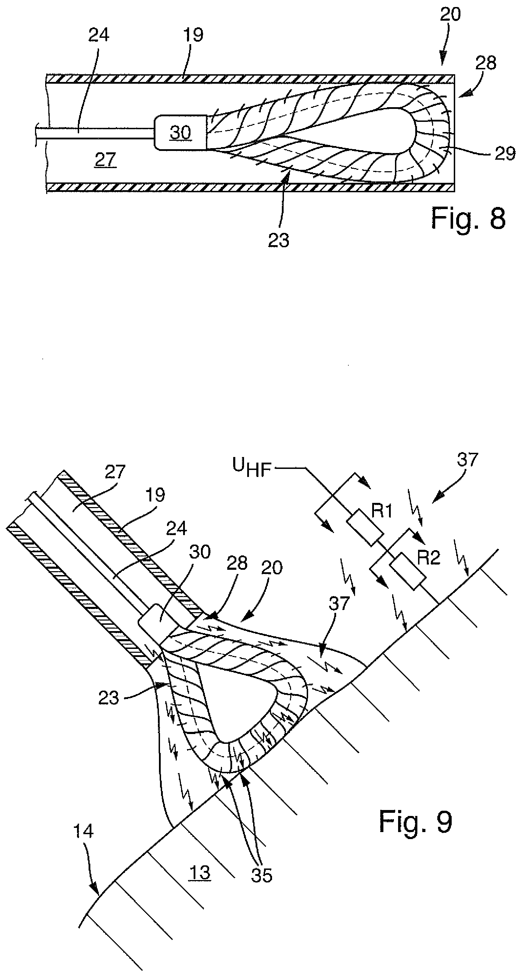

Die durch die Kordel 23a oder Schlinge gebildete Elektrode 23 kann an dem Schaft 18 in vorgegebener fixer Position gehalten sein. Es ist aber auch möglich, die Elektrode 23 beweglich anzuordnen. In diesem Fall kann die Elektrode 23 durch entsprechende Axialbewegung entweder in die in Figur 7 veranschaulichte exponierte Position oder in die in Figur 8 veranschaulichte zurückgezogene Position überführt werden. In zurückgezogener Position ist die Elektrode 23 teilweise oder vollständig in dem Lumen 27 gehalten. Die z.B. als Schleife ausgebildete Elektrode 23 kann in ausgefahrenem Zustand gemäß Figur 7 eine Breite aufweisen kann, die größer ist als der Innendurchmesser des Schlauchs 19 und somit des Lumens 27. Dank ihrer Flexibilität kann die Elektrode 23 in der Breite komprimiert werden, sodass sie gemäß Figur 8 in das Lumen 27 passt.

Die Funktion des insoweit beschriebenen Instruments 15 wird nachfolgend am Beispiel sowie unter Bezugnahme auf Figur 9 erläutert:

Zur Koagulation der Gewebeoberfläche 14 wird das Instrument 15 mit seinem distalen Ende 20 in die Nähe der Gewebeoberfläche 14 verbracht. Über das Lumen 27 strömt Argon in distaler Richtung und umfließt die Elektrode 23. Diese steht über die Leitung 24 mit dem Hochfrequenzgenerator 22 in Verbindung und erhält über diesen eine Wechselspannung von mehreren 100 Volt mit einer Frequenz deutlich über 100 kHz. Die Elektrode 23 kann dabei aus der Öffnung 28 herausragend positioniert sein und zumindest punktuell die Gewebeoberfläche 14 berühren. Jedoch weist sie einen entlang ihrer Länge einen Widerstandsbelag auf, der, der in Figur 9 nebenstehend durch Widerstände R1, R2 symbolisch eingetragen ist, an denen die von dem Generator 22 abgegebene Spannung U_{HF} anliegt.

Die Elektrode 23 kann durch direkten Kontakt zu der Gewebeoberfläche 14 dort einen Koagulationseffekt hervorrufen. Dabei kann durch das Lumen 27 herbeiströmendes Argon die Elektrode 23 schützend umhüllen und dazu führen, dass sich von der Elektrode 23 ausgehend Funkenplasma bildet, das die Gewebeoberfläche 14 berührt. In Abhängigkeit von der Größe der Berührungsfläche zwischen der Elektrode 23 und der Gewebeoberfläche 14 und dem Andruck variiert der durch die Elektrode 23 gebildete elektrische Widerstand zwischen der Leitung 24 und der Gewebeoberfläche 14 zu größeren oder kleineren Werten. Gerade durch den nichtmetallischen Aufbau der Elektrode 23 kann der von der Elektrode 23 an den Kontaktstellen zu dem Gewebe 13 übergehende Strom beschränkt werden, sodass zusätzlich zu der Kontaktkoagulation eine Plasmabildung, zum Beispiel an der Fassung 30 oder Teilen der Elektrode 23, stattfindet. Das Plasma ist in Figur 9 sowohl in der körperlichen Darstellung als auch in der nebenstehenden schematisch elektrischen Darstellung durch gezackte Pfeile 37 veranschaulicht.

Die Kohlenstofffasern 29 können zu einer ausgeprägten elektrischen Anisotropie der Elektrode 23 beitragen. Beispielsweise können die von der Kordel 23a oder auch den Elektroden nach Figur 3 oder 6 wegstehenden Enden 35 der Kohlenstofffasern als bevorzugte Stromaustrittspunkte dienen, von denen Plasmafäden ausgehen. Insbesondere aber verhindert die Ausbildung der Elektrode 23 aus Kohlenstofffasern 29 ein Ankleben oder Haften der Elektrode 23 an der Gewebeoberfläche 14 und somit eine Verletzung derselben. Ob die Elektrode 23 als Pinsel 31, als Spatel 36 oder als ovaler Körper 33 ausgebildet ist - jedenfalls tragen die Kohlenstofffasern 29 zur gleichmäßigen Stromverteilung auf der Gewebeoberfläche 14 und zur Verhinderung des Anhaftens der Elektrode 23 an derselben bei. Außerdem kann die hohe Wärmeleitfähigkeit der Kohlenstoffasern dazu genutzt werden, die Temperatur der Elektrode relativ niedrig zu halten, wodurch ebenfalls die Neigung des Gewebes zum Anhaften an der Elektrode gemindert werden kann.

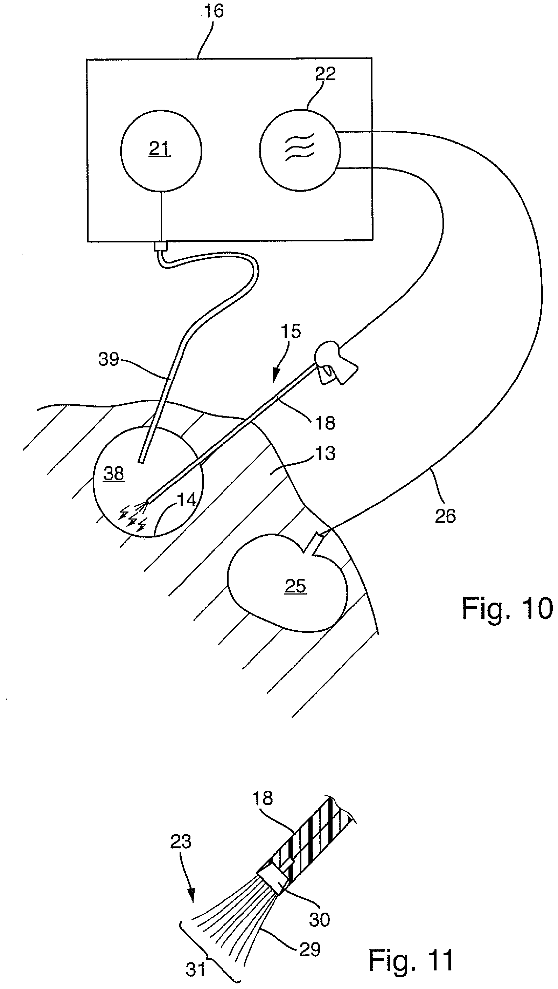

Eine weitere Ausführungsform der Erfindung ergibt sich aus den Figuren 10 und 11:
Bei der Ausführungsform nach Figur 10 weist das Instrument 15 einen steifen Schaft 18 auf, der wie bei den vorstehenden Ausführungsformen ein gasführendes Lumen aufweisen kann. Es ist jedoch auch möglich, auf eine Gaszuführung durch den Schaft 18 zu verzichten und eine entsprechende Körperhöhle 38 über einen gesonderten Zugang 39 mit Gas aus der Gasquelle 21 zu versorgen. Die Elektrode 23 kann dabei nach jeder der vorstehend beschriebenen Arten ausgebildet sein. Figur 11 veranschaulicht beispielhaft die Ausbildung als Pinsel 31. Von den freien Enden der Kohlenstofffasern 29 ausgehend, können sich, wenn die Körperhöhle 38 mit schützendem Gas, beispielsweise Argon gefüllt ist, Plasmafäden zu der Gewebeoberfläche 14 ausbilden. Der Behandler kann nun den Pinsel 31 mit oder ohne Berührung der Körpergewebeoberfläche 14 über große Areale derselben führen und diese koagulieren.

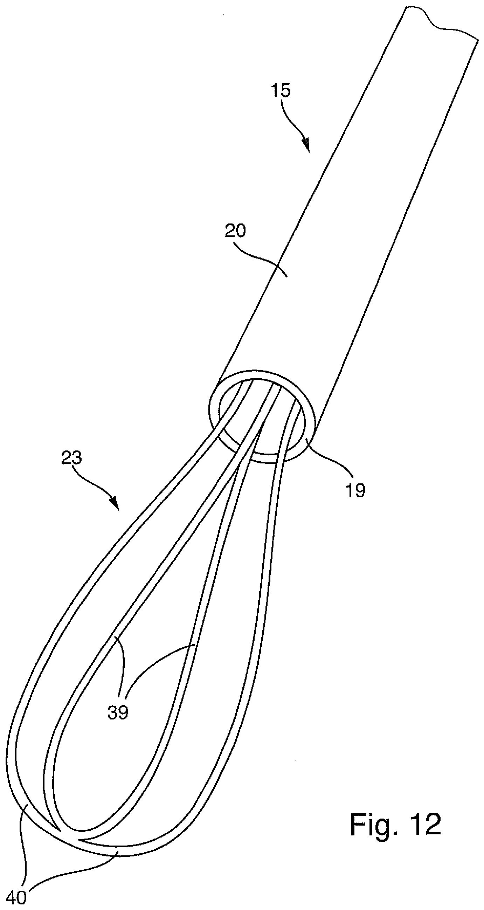

An dem Instrument sind zahlreiche weitere Variationen möglich. Beispielsweise veranschaulicht Figur 12 das Instrument 15 weitgehend entsprechend der in den Figuren 7 bis 9 beschriebenen Ausführungsformen. Während jedoch die Elektrode 23 bei der Ausführungsform nach den Figuren 7 bis 9 als einfache Schlinge aufgebaut ist, ist es möglich, die Elektrode auch mit mehreren Schlingen, beispielsweise zwei Schlingen 39, 40 auszubilden, die um 90° zueinander positioniert sind. An ihrem proximalen Ende kann diese Elektrode 23 mit einem sich durch den Schlauch 19 erstreckenden Leiter elektrisch verbunden sein. Die Schlingen 39, 40 können wiederum als Kordeln, gedrehte oder geflochtene Fäden aus Kohlenstofffasern ausgebildet sein, die mit dem sich durch den Schlauch 19 erstreckenden Leiter, beispielsweise durch Krimpen verbunden sind. Hinsichtlich der Arbeitsweise und Funktion des Instruments 15 nach Figur 12 gelten die vorstehenden Ausführungen unter Nutzung der bereits eingeführten Bezugszeichen entsprechend. In den Schlingen 39, 40 können ein oder mehrere Drähte eingearbeitet sein, um die Steifigkeit der Elektrode 23 zu erhöhen und dieser federnde Rücksprungeigenschaften zu geben. Diese Drähte können ähnlich wie der Draht 30a in Figur 7 zentral durch die jeweiligen Schenkel der Schlingen 39, 40 gehen.

Bei allen vorstehend beschriebenen Ausführungsformen kann die aus Kohlenstoff bestehende Elektrode durch eine Zuleitung aus Metall elektrisch kontaktiert werden. Insbesondere ist es vorteilhaft, wenn das Element durch das die Elektroden gehalten werden, zum Beispiel eine Krimphülse und eventuell auch die elektrische Zuleitung eine erhöhte Wärmeleitfähigkeit haben. Zum Beispiel kann die Zuleitung aus Kupfer oder auch aus mit einer wärmeleitenden Beschichtung versehenen Edelstahldraht hergestellt sein.

Bei einem erfindungsgemäßen Instrument 15 zur chirurgischen Behandlung einer Gewebeoberfläche 14 insbesondere zur Koagulation oder Ablation derselben, dient eine aus Kohlenstofffasern 29 bestehende oder wenigstens Kohlenstofffasern 29 aufweisende Elektrode 23 zur Stromzuleitung zu der Gewebeoberfläche 14. Infolge der Anisotropie der elektrischen Leitfähigkeit der Kohlenstofffasern 29 oder der ausgebildeten Elektrode 23 kann eine großflächige und gleichmäßige Stromverteilung erreicht werden. Und zwar dies sowohl in direkter Kontaktkoagulation, als auch in gemischter Koagulation bei Plasmaausbildung unter zumindest teilweiser Berührung der Gewebeoberfläche 14 durch die Elektrode 23. Durch die Ausbildung der Elektrodenoberfläche durch Kohlenstofffasern 29 und insbesondere deren hoher Wärmeleitfähigkeit wird ein Anhaften der Elektrode (23) an der Gewebeoberfläche (14) wirksam unterbunden.

### Bezugszeichen:

- 12: Einrichtung zur Gewebekoagulation
- 13: Gewebe
- 14: Gewebeoberfläche
- 15: Instrument
- 16: Gerät
- 17: Stecker
- 18: Schaft
- 19: Schlauch
- 20: distales Ende des Instruments
- 21: Gasquelle
- 22: HF-Generator
- 23: Elektrode
- 23a: Kordel
- 24: Leitung zu der Elektrode 23
- 25: Neutralelektrode
- 26: Leitung zu der Neutralelektrode 25
- 27, 27a: Lumen
- 28: Öffnung
- 29: Kohlenstofffaser
- 30: Fassung
- 30a: Draht
- 31: Pinsel (Figur 2)
- 32: Pfeil
- 33: Körper (Figur 3)
- 34: Kunststoffmatrix
- 35: Enden der Kohlenstofffasern
- 36: Spatel
- 37: gezackte Pfeile
- 38: Körperhöhle
- 39, 40: Schlingen

## Patentansprüche

1. Instrument (15) zur Behandlung lebenden Gewebes (13),
mit einem länglichen Schaft (18), der an einem distalen Ende (20) eine Elektrode (23) aufweist, die mit einer an einen elektrischen Generator (22) angeschlossenen oder anschließbaren elektrischen Leitung (24) verbunden ist,
**dadurch gekennzeichnet,**
**dass** die Elektrode (23) Kohlenstofffasern (29) aufweist.

2. Instrument nach Anspruch 1, **dadurch gekennzeichnet, dass** der längliche Schaft (18) ein Schlauch (19) oder Rohr ist, der oder das mindestens ein Lumen (27) aufweist, welches an eine Gasquelle (21) angeschlossen oder anschließbar ist und welches an einem distalen Ende (20) des Schlauchs (19) oder Rohrs eine Auslassöffnung (28) aufweist.

3. Instrument nach Anspruch 2, **dadurch gekennzeichnet, dass** die Elektrode (23) in oder an der Auslassöffnung (28) angeordnet ist.

4. Instrument nach einem der vorstehenden Ansprüche, **dadurch gekennzeichnet, dass** die Elektrode (23) in oder an dem Schaft (18) beweglich gelagert ist.

5. Instrument nach einem der Ansprüche 2 bis 4, **dadurch gekennzeichnet, dass** die Elektrode (23) vollständig in das Lumen (27) einziehbar ausgebildet ist.

6. Instrument nach einem der vorstehenden Ansprüche, **dadurch gekennzeichnet, dass** die Elektrode (23) als Schlinge ausgebildet ist.

7. Instrument nach einem der vorstehenden Ansprüche, **dadurch gekennzeichnet, dass** die Elektrode (23) flexibel ausgebildet ist.

8. Instrument nach einem der vorstehenden Ansprüche, **dadurch gekennzeichnet, dass** wenigstens einige der Kohlenstoffasern (29) ein bewegliches freiliegendes Ende (35) aufweisen.

9. Instrument nach einem der vorstehenden Ansprüche, **dadurch gekennzeichnet, dass** wenigstens einige der Kohlenstoffasern (29) ein in der Elektrode (23) fixiertes Ende aufweisen.

10. Instrument nach einem der vorstehenden Ansprüche, **dadurch gekennzeichnet, dass** die Kohlenstofffasern (29) in Form eines Pinsels (31) gefasst sind.

11. Instrument nach einem der vorstehenden Ansprüche, **dadurch gekennzeichnet, dass** die Kohlenstofffasern (29) als Kordel (23a) ausgebildet ist.

12. Instrument nach einem der vorstehenden Ansprüche, **dadurch gekennzeichnet, dass** die Leitung (24) sich von der Elektrode (23) bis zu einem proximalen Anschlussmittel (17) erstreckend ausgebildet ist.

13. Instrument nach einem der Ansprüche 2 bis 12, **dadurch gekennzeichnet, dass** die Leitung (24) in dem Lumen (27) angeordnet ist.

14. Instrument nach einem der Ansprüche 2 bis 13, **dadurch gekennzeichnet, dass** der Schaft (18) mehrere Lumen (27, 27a) aufweist, die von einem proximalen Ende des Schafts (18) zu dem distalen Ende (20) führen.

15. Instrument nach Anspruch 14, **dadurch gekennzeichnet, dass** die Lumen (27, 27a) mit einem Anschlussmittel (17) verbunden sind, das zur Verbindung der Lumen (27, 27a) mit unterschiedlichen Fluidversorgungsquellen (21, 21a) eingerichtet ist.
